# EUROPÄISCHE PATENTSCHRIFT

(11) **EP 1 887 355 B1**
(45) Veröffentlichungstag und Bekanntmachung des Hinweises auf die Patenterteilung: **27.09.2017**
(21) Anmeldenummer: 06016068.6
(22) Anmeldetag: 02.08.2006
(51) Int. Cl.: B01L 3/00, A61B 5/15, A61B 5/145, A61B 5/151

(54) **BESCHICHTUNGSVERFAHREN FÜR EIN MIKROFLUIDIKSYSTEM.**
COATING METHOD FOR A MICROFLUIDIC SYSTEM.
PROCÉDÉ DE REVÊTEMENT D'UN SYSTÈME MICROFLUIDIQUE.

(43) Veröffentlichungstag der Anmeldung: 13.02.2008
(73) Patentinhaber: F. Hoffmann-La Roche AG, 4070 Basel (CH); Roche Diabetes Care GmbH, 68305 Mannheim (DE)
(72) Erfinder: Glauser, Michael, 6343 Rotkreuz (CH); Wahl, Hans peter, 79650 Schopfheim (DE); Calasso, Irio Giuseppe, 6415 Arth (CH); Sarofim, Emad, 6332 Hagendom (CH)
(74) Vertreter: Pfiz, Thomas

(56) Entgegenhaltungen:
- EP-A- 1 612 554
- WO-A-01/72220
- WO-A1-2006/063181
- DE-A1- 10 128 460
- DE-T2- 60 011 429
- US-A1- 2005 084 681
- US-A1- 2005 176 678

## Beschreibung

Die Erfindung betrifft ein Verfahren zum Aufbringen einer hydrophilen Oberflächenbeschichtung auf ein solches Mikrofluidiksystem.

Mikrofluidiksysteme werden speziell in Analysesystemen für Körperflüssigkeiten, bspw. in Blutzuckermessgeräten, mit denen Diabetiker selbst ihren Blutzuckerspiegel kontrollieren können, verwendet. Das System beinhaltet ein Stechorgan, welches mit dem Mikrokanal zum kapillaren Transport einer Körperflüssigkeit verbunden ist. Die Flüssigkeit wird vom Mikrokanal zu einem Sammelbereich transportiert, um die jeweilige Messgröße, bspw. den Blutzuckergehalt, getrennt vom Stechort erfassen zu können. Derartige Systeme sind beispielsweise in der WO2006/021361 und EP 1671585 offenbart, auf deren Inhalt vor allem hinsichtlich der Herstellung solcher Mikrostrukturen ausdrücklich Bezug genommen wird. Der Mikrokanal und ggf. das Stechorgan wird aus einem biokompatiblen, also insbesondere gegen die Körperflüssigkeit inerten Material gefertigt, das sowohl mechanisch beanspruchbar als auch leicht zu reinigen und zu sterilisieren sein muss. Besonders geeignet ist hierfür Chirurgenstahl (316L), der allerdings zu wenig hydrophil ist, um einen kapillaren Transport von wässrigen Körperflüssigkeiten durch den Mikrokanal zu gestatten. Aus diesem Grund werden dauerhafte und stabile hydrophile Oberflächenbeschichtungen gesucht, die zudem biokompatibel (insbesondere nach ISO 10993) sind. Die Oberflächenbeschichtung muss außerdem die Befüllung des Mikrokanals innerhalb einer sehr kurzen Zeit, vorzugsweise weniger als zwei Sekunden, erlauben, damit die Körperflüssigkeit schnell gesammelt werden kann. Dies ist vor allem wichtig für das Sammeln von Blut zur Bestimmung des Blutzuckergehalts, da innerhalb einer solch kurzen Zeit noch nicht mit Gerinnungsreaktionen zu rechnen ist, so dass der Benutzer sehr schnell ein Messresultat erhält. Die Oberflächenbeschichtung muss zudem mit mindestens einer Sterilisationsmethode kompatibel sein, wobei die Sterilisation mittels Elektronenstrahl (β-Strahlung) bevorzugt ist, weil diese Sterilisationsmethode schonend, kontaktfrei und fokussierbar ist.

Schließlich ist eine ausreichende Langzeitstabilität der Oberflächenbeschichtung erwünscht. Dies stellt ein Problem dar, weil hydrophile Beschichtungen in der Regel hochenergetische Oberflächen aufweisen. Dies ist thermodynamisch ungünstig, weil die Oberfläche bestrebt ist, ihre hohe Energie zu reduzieren, indem die Hydrophilie reduziert wird.

Die Aufgabe der vorliegenden Erfindung besteht somit darin, ein Beschichtungsverfahren für ein Mikrofluidiksystem der o.g. Art bereitzustellen, um dieses auf möglichst einfache Weise mit einer langzeitstabilen, biokompatiblen, hydrophilen Oberflächenbeschichtung zu versehen. Zur Lösung dieser Aufgabe werden die im unabhängigen Anspruch angegebenen Merkmalskombinationen vorgeschlagen. Vorteilhafte Ausgestaltungen und Weiterbildungen der Erfindung ergeben sich aus den abhängigen Ansprüchen.

Demnach wird vorgeschlagen, den Mikrokanal mit einer Oberflächenbeschichtung bestehend aus mindestens einer hydrophilen Substanz ausgewählt aus der Gruppe Polyacrylsäure, Polyacrylat, Dextransulfat, Chondroitinsulfat zu versehen. Ein Mikrofluidiksystem mit einer solchen hydrophilen Oberflächenbeschichtung weist überraschenderweise alle geforderten Eigenschaften auf. Die Oberflächenbeschichtung ist biokompatibel, dauerhaft hydrophil und nach dem Aufbringen auf die zu beschichtende Oberfläche sterilisierbar. Sie bewirkt zuverlässig schnelle Füllzeiten des Mikrokanals und weist eine große Kapazität gegen Verschmutzung auf.

Als Beschichtungsmaterial sind sowohl einfache lineare als auch ganz oder teilweise vernetzte Polyacrylsäuren bzw. Polyacrylate geeignet. Polyacrylsäure und Polyacrylate, d.h. die neutralisierten Derivate von Polyacrylsäure, sind bisher in der Pharmaindustrie als Geliermittel bei der Formulierung von Salben bekannt, wobei Wirkstoffe in eine Hydrogelmatrix eingebettet werden. Polyacrylsäuren finden ferner als Superabsorber Verwendung. Es hat sich nun in unerwarteter Weise herausgestellt, dass sich Oberflächen, insbesondere aus biokompatiblem Material wie Edelstahl oder anderen inerten Metallen, dauerhaft direkt mit Polyacrylsäuren und/oder mit ihren Salzen beschichten lassen und die resultierende Beschichtung praktisch optimal die mit dem erfindungsgemäßen Mikrofluidiksystem verbundenen Anforderungen erfüllt. Dabei kann die Polyacrylsäure bzw. ihr Salz direkt aus einer Dispersion auf die zu beschichtende Oberfläche aufgebracht werden. Nach Entzug der Flüssigkeit entsteht eine transparente Schicht mit allen erforderlichen Eigenschaften. Das erfindungsgemäße Verfahren erlaubt somit die einfache und kostengünstige Beschichtung insbesondere von Massenprodukten wie disposibler Mikrofluidiksysteme, zumal nur sehr wenig Polyacrylsäure benötigt wird.

Alternativ kann die Oberfläche vorzugsweise durch eine Eintauchbeschichtung mittels Dextransulfat oder Chondroitinsulfat hydrophilisiert werden. Dabei bildet sich eine Einfachschicht, welche durch eine ionische Bindung irreversibel und stabil haftet. Bei beiden Zuckersulfaten handelt es sich um Substanzen, die biologisch nicht aktiv sind und daher auch für in vivo Anwendungen (Gewinnung einer Körperflüssigkeit durch Stechen) unbedenklich sind. Beide Substanzen erfüllen die nach der Norm ISO 10993 geforderten Biokompatibilitäts-Kriterien.

Bevorzugt ist ein Stechorgan mit dem Mikrokanal verbunden, welches ebenfalls mit der Oberflächenbeschichtung versehen wird. Aufgrund der kleinen Abmessungen bietet es sich an, dass der Mikrokanal und/oder das Stechorgan über seine gesamte Oberfläche mit der Oberflächenbeschichtung versehen sind. Die zu beschichtende Oberfläche des Mikrokanals bzw. des Stechorgans besteht vorzugsweise aus einem biokompatiblen Material, insbesondere Edelstahl. Auch für die Massenfertigung als Verbrauchsmittel (Disposables) ist es günstig, wenn der Mikrokanal als halboffener rillenförmiger Kapillarkanal an einem flachen Träger ausgebildet ist. Für eine weitergehende Integration ist es von Vorteil, wenn der Mikrokanal mit einem Testfeld zum Nachweis eines Analyten in der Flüssigkeit verbunden oder verbindbar ist.

Zur Herstellung einer solchen hydrophilen Oberflächenbeschichtung wird vorgeschlagen, dass mindestens eine Substanz ausgewählt aus der Gruppe Polyacrylsäure, Polyacrylat, Dextransulfat, Chondroitinsulfat in einer Flüssigkeit, insbesondere Wasser, in Kontakt mit einer zu beschichtenden Oberfläche gebracht und anschließend die Flüssigkeit entfernt wird, wobei die (zuvor formlose) Substanz eine hydrophile Schicht bildet. Die zu beschichtende Oberfläche wird vor dem Aufbringen der Oberflächenbeschichtung mittels Sauerstoff-Niederdruckplasma-Verfahren, Corona Discharge Verfahren und/oder Säureätzungsverfahren gereinigt, die dem Fachmann als solche bekannt sind und zusätzlich den Vorteil haben, dass sie die zu beschichtende Oberfläche aktivieren.

Eine Ausführung sieht vor, dass Polyacrylsäure in einer Flüssigkeit, insbesondere Wasser, dispergiert wird, und die erhaltene Dispersion auf die zu beschichtende Oberfläche aufgebracht wird. Hierbei kann es von Vorteil sein, die erhaltene Dispersion mit einer Base zu neutralisieren. Damit kann die Dispersion auf den pH-Wert der zu untersuchenden Flüssigkeiten, bspw. des Blutes gebracht werden, so dass sie mit der zu untersuchenden Flüssigkeit besonders verträglich ist.

Die Dispersion kann vor dem Aufbringen verdünnt werden, bspw. mit der Dispergierflüssigkeit, welche vorzugsweise Wasser ist. Auf diese Weise kann die Viskosität der Dispersion an das jeweilige Verfahren angepasst werden, mit dem sie auf die zu beschichtende Oberfläche aufgebracht wird. Geeignet sind bspw. Pipettieren, Sprayen, Dip-Coating-Verfahren oder Spin-Coating-Verfahren. Nach dem Aufbringen der Dispersion wird durch ein Trocknungsverfahren, bspw. Erhitzen, die Dispergierflüssigkeit entzogen, so dass die gewünschte Oberflächenbeschichtung entsteht.

Gemäß einer weiteren bevorzugten Variante des Beschichtungsverfahrens wird die zu beschichtende Oberfläche in eine Dextransulfat und/oder Chondroitinsulfat enthaltende wässrige Lösung eingetaucht, wobei die so erhaltene Schicht insbesondere durch eine ionische Bindung fest an die Oberfläche gebunden wird. Um überschüssige Substanzen zu entfernen, ist es günstig, wenn die Oberfläche nach dem Aufbringen der hydrophilen Schicht vorzugsweise in einem Wasserbad gewaschen wird.

Im Folgenden wird die Erfindung anhand des in der Zeichnung schematisch dargestellten Ausführungsbeispiels näher erläutert. Es zeigen
- Fig. 1: ein Mikrofluidiksystem in Form eines Mikrosamplers in einer schaubildlichen Darstellung;
- Fig. 2: einen nicht maßstäblich vergrößerten Querschnitt des Mikrosamplers nach Fig. 1;
- Fig. 3: ein Testplättchen als Mikrofluidiksystem in der Draufsicht;
- Fig. 4: ein Messdiagramm einer Stabilitätsstudie mit Testplättchen nach Fig. 3;
- Fig. 5: ein weiteres Testplättchen in der Draufsicht;
- Fig. 6 und 7: Messdiagramme einer Stabilitätsstudie mit Testplättchen nach Fig. 5.

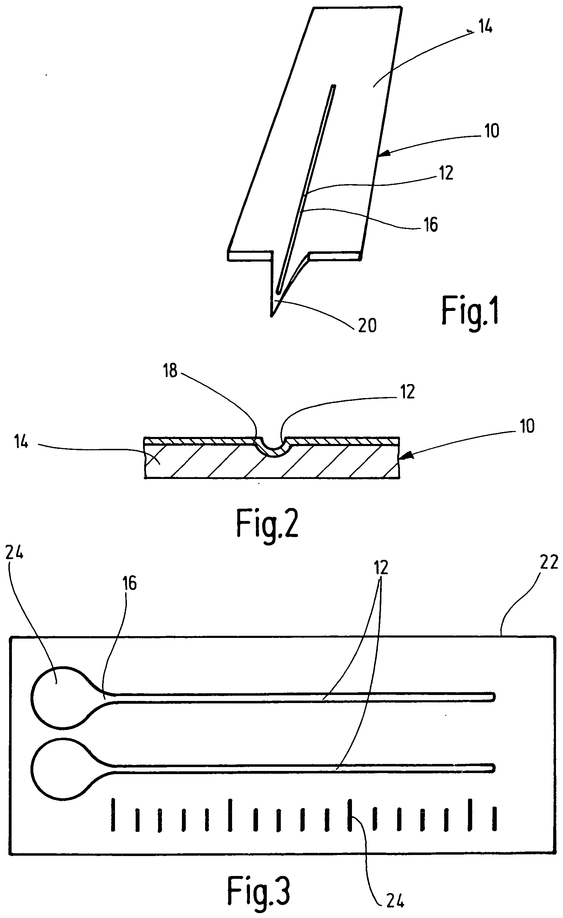

Das in Fig. 1 und 2 gezeigte Mikrofluidiksystem 10 bildet als so genannter Mikrosampler ein disposibles Element zum Aufnehmen und Bereitstellen von Körperflüssigkeit, speziell Blut oder Gewebeflüssigkeit für Analysezwecke, insbesondere Blutzuckermessungen. Hierfür ist ein Mikrokanal bzw. Kapillarkanal 12 vorgesehen, der ohne externe Aktuation allein durch Kapillarwirkung einen Flüssigkeitstransport von einer distalen Aufnahmestelle zu einer proximalen Zielstelle ermöglicht. Der Kanal 12 ist rillenförmig in einen flachen Träger 14 aus Edelstahlblech eingebracht, beispielsweise durch Maskenätzen oder Laserschneiden. An seiner durch die Oberseite des Trägers 14 gebildeten Oberfläche 16 ist der Kanal 12 mit einer hydrophilen Oberflächenbeschichtung 18 versehen, die eine schnelle und vollständige Befüllung gewährleistet.

Das distale Ende des Kanals 12 liegt im Bereich eines beispielsweise in einen Finger einstechbaren Stechorgans 20, während das proximale Kanalende in fluidische Verbindung mit einem nicht gezeigten Testfeld gebracht werden kann, welches eine geeignete Testchemie zum Glukosenachweis enthält. Zu diesem Zweck kann der Mikrosampler 10 in ein auch von Laien bedienbares Handgerät eingesetzt werden, wie es für die Analyse von Teststreifen bereits bekannt ist. Entscheidend für den Einsatz sind neben Biokompatibilität und Sterilisierbarkeit eine möglichst dauerhafte Erhaltung der hydrophilen Beschichtung 18.

### BEISPIEL 1

Für eine Langzeitstabilitätsstudie wurden gemäß Fig. 3 Testplättchen 22 aus Chirurgenstahl mit zwei 16mm langen und 0,2mm breiten halboffenen Mikrokanälen 12 versehen. Am Anfang der Kanäle 12 wurde jeweils eine Auftragungsstelle 24 für Blut als erweiterte Vertiefung geschaffen. Die Testplättchen 22 wurden auf die unten beschriebene Weise vollflächig beschichtet. Auf diese Weise wurde auch die vertiefte Oberfläche 16 der Mikrokanäle 12 und der Auftragsstelle 14 mit einer hydrophilen Oberflächenbeschichtung 18 versehen. An einer Längsseite der Stahlplättchen 22 wurde ein Strichmaßstab 24 eingebracht.

Die Beschichtung beruht auf dem Grundprinzip, mit der erforderlichen Reinheit kommerziell erhältliche, also fertig polymerisierte Polyacrylsäuren (PAA), die traditionell als Gelierungsmittel in der Pharmaindustrie und als Superabsorber eingesetzt werden, als Ausgangsmaterial für die Herstellung einer hydrophilen Oberflächenschicht einzusetzen.

Hierfür wird zunächst eine Polyacrylsäure, im Ausführungsbeispiel das Produkt der Firma Sigma-Aldrich Nr. 43,532-5 "Poly(Acrylic acid), Potassium salt, ligh cross-linked" in einer für den jeweiligen Anwendungsfall geeigneten Flüssigkeit, im Ausführungsbeispiel Wasser, dispergiert. Statt einer quervernetzten Polyacrylsäure kann selbstverständlich auch eine nicht vernetzte, lineare Polyacrylsäure verwendet werden. Durch das Dispergieren wird eine viskose Dispersion oder Gelmatrix erhalten.

Diese Gelmatrix kann dann mit einer Base neutralisiert werden, um den pH-Wert auf einen gewünschten pH-Wert einzustellen. Wenn bspw. ein Mikrofluidiksystem zum Transport einer Körperflüssigkeit beschichtet werden soll, kann die Dispersion oder Gelmatrix auf den pH-Wert der zu untersuchenden Flüssigkeit, im Ausführungsbeispiel des Blutes, eingestellt werden. Hierfür sind verschiedenste Basen geeignet, insbesondere Natronlauge, Kalilauge oder basische Aminosäuren. Dabei entsteht ein Polyacrylsäuresalz oder Polyacrylat. Die Wahl der geeigneten Base hängt von den jeweiligen Anforderungen an die Biokompatibilität und Toxikologie der herzustellenden Oberflächenbeschichtung ab. Im Ausführungsbeispiel wurde der pH-Wert mit KOH auf 7,0 eingestellt.

Während der Neutralisierung nimmt die Viskosität der Dispersion stark zu, weil sich die einzelnen Stränge der Polyacrylsäure aufgrund der elektrostatischen Abstoßung entfalten. Dies hat jedoch den Vorteil, dass auch nach einer Neutralisierung die Dispersion durch Verdünnen problemlos auf die gewünschte Viskosität eingestellt werden kann, die für das jeweils gewählte Applikationsverfahren am besten geeignet ist. Im Ausführungsbeispiel wurde die neutralisierte Dispersion mit Wasser so stark verdünnt, dass eine sehr niederviskose Dispersion erhalten wurde. Zusätzlich wurden die langen Polyacrylsäure-Fäden mechanisch zerkleinert.

Die zu beschichtende Oberfläche 16 wurde vor dem Beschichten mit dem an sich bekannten Sauerstoff-Niederdruckplasma-Verfahren gereinigt und aktiviert. Anschließend wurde das Testplättchen 22 in die sehr niedrigviskose Dispersion getaucht. Nach dem oberflächlichen Abtrocknen wurde das Wasser aus der Dispersion durch Erhitzen auf 80°C entfernt. Es blieb eine eingetrocknete Polyacrylatschicht zurück, die fest auf dem Testplättchen haftete. Die Schicht war transparent und mit bloßem Auge nicht zu erkennen, so dass sich vermutlich ein hauchdünner Film aus Polyacrylat gebildet hatte. Diese transparente Schicht wirkt stark hydrophil, also wasseranziehend.

Die Testplättchen 22 wurden nach dem Beschichten während 3h bei 130°C hitzesterilisiert und dann in Glas verpackt, welches ein sehr reines Verpackungsmaterial darstellt.

Die so behandelten und verpackten Testplättchen 10 wurden 3 Monate bei 35°C gelagert. Diese Lagerbedingungen stellen ein Modell für eine Lagerung über 2 Jahre bei Raumtemperatur dar. In regelmäßigen Abständen wurden Testplättchen 22 entnommen und sowohl auf die Befülllänge als auch auf die Befüllzeit der Mikrokanäle 12 getestet.

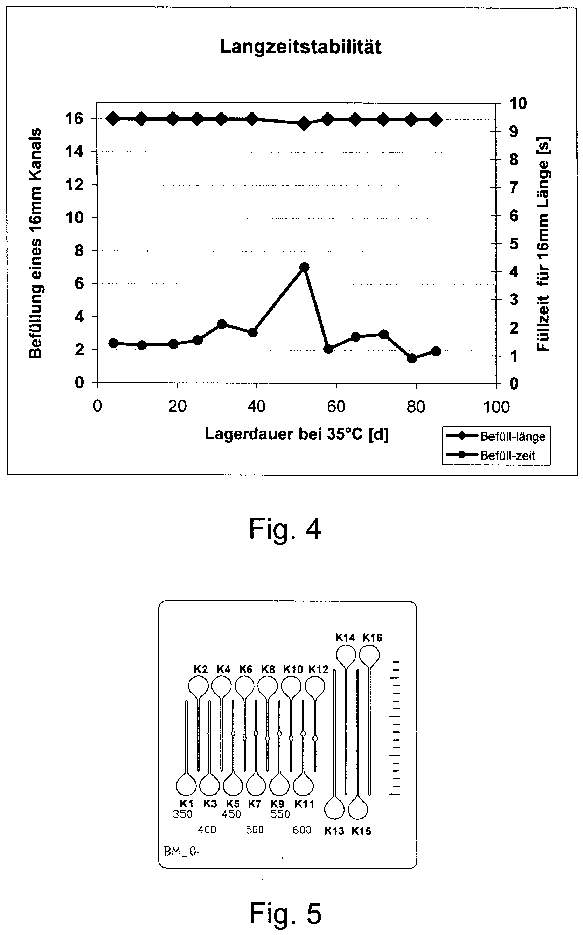

Gemäß Fig. 4 zeigte sich dabei, dass die Mikrokanäle 12 aller Proben vollständig befüllt wurden. Die durchschnittliche Befüllzeit betrug um die zwei Sekunden, wobei nach langer Lagerzeit sogar eine leichte Verbesserung zu beobachten war. Dies ist ein sehr guter Wert; in Kontaktwinkel ausgedrückt, entspricht die zu befüllende Strecke von 16 mm einem Kontaktwinkel von unter 10°. Die Beschichtung erwies sich somit als sehr sta-bil, und ihre Hydrophilie ließ während der Lagerzeit nicht nach.

Ferner wurde geprüft, ob sich die beschriebene Beschichtung mit Elektronenstrahlen (β-Strahlen) sterilisieren lässt. Dazu wurden Plättchen aus Chirurgenstahl mit einem 16 mm langen Mikrokanal versehen und wie oben beschrieben beschichtet. Anschließend wurde die Hälfte der Plättchen in PET-Beutel verpackt und mit β-Strahlung (10MeV, 25kGy) sterilisiert. Danach wurden die jeweilige durchschnittliche Befülllänge und die jeweilige durchschnittliche Befülldauer der bestrahlten bzw. der nicht bestrahlten Plättchen bestimmt und verglichen. Das Ergebnis ist in Tabelle 1 dargestellt.

**Tabelle 1**

| | ∅ Befülllänge | ∅ Befülldauer | Standardabweichung der Befülldauer |
|---|---|---|---|
| Sterilisiert | 16,0 mm | 1,65 sek. | 0,26 sek. |
| Nicht sterilisiert | 16,0 mm | 1,82 sek. | 0,56 sek. |

Diese Ergebnisse zeigen, dass die Sterilisierung mit β-Strahlen keinen nachteiligen Einfluss auf die Beschichtung hat.

Ferner wurde nach β-Sterilisation eine Biokompatibilitätsprüfung nach ISO 10993 (Test for in vitro cytotoxy ISO 10993-5, Test for irritation ISO 10993-10, Test for delayed-type hypersensivity ISO 10993-10) durchgeführt. Dazu wurde das Produkt von Sigma-Aldrich, Nr. 43,532-5 "Poly(Acrylic acid), Potassium salt, ligh cross-linked" verwendet. Vor der Biokompatibilitätsprüfung wurde dieses Produkt mit KOH auf einen pH-Wert von 7,0 gebracht. Zusätzlich wurden die langen Polyacrylsäure-Fäden mechanisch zerkleinert. Dieses Produkt wurde wie beschrieben in Wasser dispergiert und auf eine Oberfläche aus Chirurgenstahl aufgebracht. Diese Beschichtung hat alle drei Tests, die an sich bekannt sind und vorschriftsmäßig durchgeführt wurden, bestanden und kann somit der gewünschten Verwendung in Verbindung mit Mikrofluidiksystemen zugeführt werden.

### BEISPIEL 2

In einer weiteren Ausführungsform wurde ein Polyacrylat eingesetzt, welches nicht homogenisiert werden muss und als Pharma-Ausgangssubstanz eine hohe kontrollierte Reinheit besitzt. Es handelte sich hierbei um das Produkt Carbopol 971P NF Polymer der Firma Noveon, nachfolgend C971 genannt.

Bei der Herstellung der Beschichtung wurde nicht direkt eine 0,01%ige Dispersion verwendet, da diese den pH-Wert nur unmerklich verändert. Hierbei ist zu bemerken, dass die Pufferwirkung des Wassers in Kombination mit der kleinen Menge an schwacher Säure ungünstig für eine Titration ist. Aus diesem Grund wurde zunächst eine 0,1% Dispersion wie folgt hergestellt:
In ein großes Becherglas wurden 300 ml Milli-Q-Wasser vorgelegt. Mit Hilfe eines Stabmixers wurde das Wasser gerührt, so dass ein ordentlicher Strudel sichtbar war. Sodann wurden 300 mg C971 auf ein Wägepapier abgewogen und danach in das Becherglas gegeben. Nach ca. 20 Minuten Rühren waren alle größeren Agglomerate verschwunden und eine viskose Flüssigkeit entstanden (pH-Wert 3,5). Anschließend wurde 18% (w/v) KOH zugegeben, bis pH 7 erreicht wurde, wobei das Aufwickeln der PAA-Fäden etwa zu diesem Zeitpunkt stattfand. Danach erfolgte eine 10-fache Verdünnung mit Milli-Q-Wasser. Die so erhaltene 0,01% (0,1mg/ml)-Dispersion konnte ohne Filtration direkt für die Beschichtung eingesetzt werden. Es ergaben sich hervorragende Resultate für die Fülllänge und Füllzeit von Kapillaren auf Testplättchen auch nach monatelanger Lagerzeit.

### BEISPIEL 3

Dieses Ausführungsbeispiel beruht auf einer einfachen Beschichtung mit Dextransulfat oder Chondroitinsulfat mittels eines Eintauchverfahrens. Sodann können die beschichteten Oberflächen durch einfaches Waschen mit sterilem, destilliertem Wasser von dem nicht gebundenen Dextransulfat oder Chondroitinsulfat befreit werden. Nach dem anschließenden Trocknen sind die so beschichteten Mikrosampler bereit zur weiteren Verarbeitung. Bei den beiden Zuckersulfaten handelt es sich um solche Substanzen, die biologisch nicht aktiv sind.

Für Testmessungen wurden wiederum Testplättchen 30 gemäß Fig. 5 aus Stahl mit einer Dicke von 0,3mm mit 200*µ*m breiten Kapillaren K1 - K16 versehen, wobei die Kapillarlänge 9mm (K1 - K12) bzw. 16mm (K13 - K16) betrug. Die Kapillaren K1 - K12 besitzen im mittleren Teil eine unterschiedliche Verbreiterung mit einer Küvettenbreite von 350 bis 600*µ*m.

Als Beschichtungsmittel wurde eine 1% (w/v) Dextransulfat- bzw. Chondroitinsulfatlösung eingesetzt (Dextransulfat: Produktnummer 31395 von Sigma-Aldrich; Chondroitinsulfat: Produktnummer 27042 von Sigma-Aldrich; Lösungsmittel: Milli-Q Wasser).

Die Testplättchen 30 wurden gemäß den folgenden Prozessschritten beschichtet:
1. Plasmaaktivierung: 1 min Vakuum, 1 min Vakuum und O₂-Spülen, 2 min Plasma.
2. Beschichten: 10 min in Behälter mit Dextransulfat- bzw. Chondroitinsulfatlösung.
3. Waschen: Die beschichteten Plättchen wurden aus den Lösungen genommen, abgetropft und dann in Wasserbad auf einem Shaker während 5 min gewaschen. Überschüssiges adsorbiertes Dextransulfat und Chondroitinsulfat wurde weggewaschen und es verblieb eine Einfachschicht aus Dextransulfat bzw. Chondroitinsulfat.
4. Trocknen: Die beschichteten Microsampler wurden 10 min im Ofen bei 80°C getrocknet (alternativ: Trockenblasen im N₂ Gasstrom).
5. Verpacken: Die beschichteten Plättchen wurden in einem Mylarbeutel eingeschweisst.
6. Sterilisation: Die so hergestellten Plättchen wurden mittels Elektronenstrahl sterilisiert.

Der Nachweis einer erhöhten Hydrophilie der Testplättchen 30 konnte über Kontaktwinkelmessungen erbracht werden. Unbehandelte Plättchen haben einen Kontaktwinkel mit destilliertem Wasser von ca. 80°, während die mit Dextransulfat und auch mit Chondroitinsulfat beschichteten Plättchen einen Kontaktwinkel von ca. 30° besitzen.

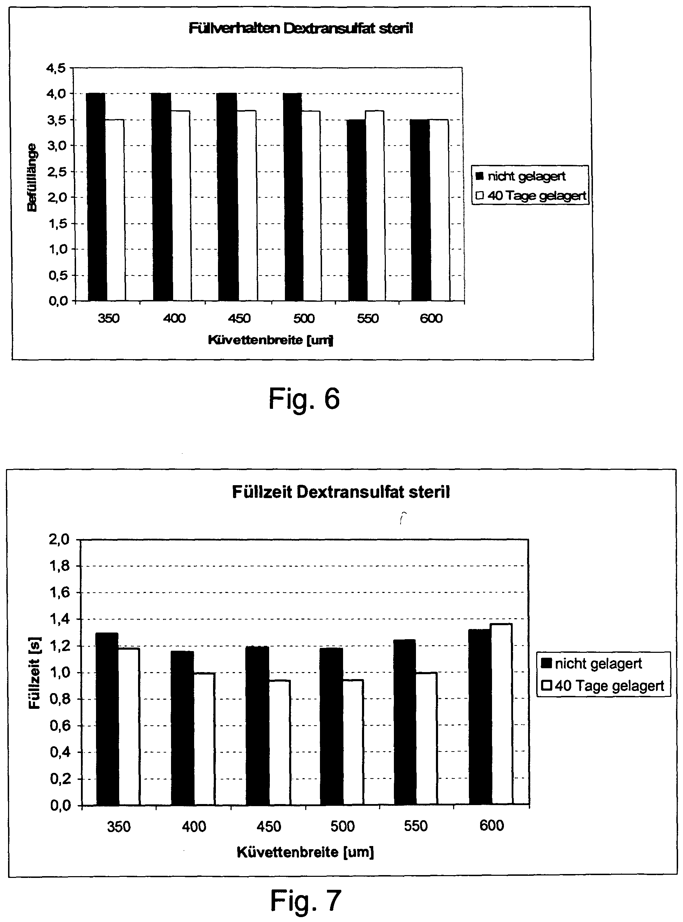

Für eine Stabilitätsbewertung wurde die Fülllänge der Kapillaren K1 - K12 gemäß Fig. 6 auch nach 40-tägiger Lagerung bestimmt, wobei ein Ordinatenwert von 4 einer Befüllung des oberen Kapillarenabschnitts über den verbreiterten Bereich hinaus entspricht. Daraus ergibt sich, dass die Hydrophilisierung eine Elektronenstrahl-Sterilisation (25 kGy, 10 MeV) übersteht und monatelang stabil bleibt. Des Weiteren spielt es eine wesentliche Rolle, wie schnell sich die Halbkapillare mit Blut füllt. Hierzu wurden gemäß Fig. 7 die entsprechenden Füllzeiten bis zu einem bestimmten Punkt in der Halbkapillare bestimmt, wobei sich auch nach Lagerung sehr gute Werte im Bereich von 1s ergaben.

## Patentansprüche

1. Beschichtungsverfahren für ein Mikrofluidiksystem, das einen Mikrokanal (12) zum kapillaren Transport einer Körperflüssigkeit für Analysezwecke aufweist, bei welchem der Mikrokanal (12) mit einer hydrophilen Schicht aus mindestens einer Substanz ausgewählt aus der Gruppe Polyacrylsäure, Polyacrylat, Dextransulfat, Chondroitinsulfat versehen wird, **dadurch gekennzeichnet, dass** die Substanz in einer Flüssigkeit, insbesondere Wasser, in Kontakt mit einer zu beschichtenden Oberfläche gebracht und anschließend die Flüssigkeit entfernt wird, wobei die zu beschichtende Oberfläche vor dem Aufbringen der Substanz mit einem Sauerstoff-Niederdruckplasma-Verfahren, Corona-Discharge-Verfahren und/oder Säureätzungsverfahren gereinigt wird.

2. Beschichtungsverfahren nach Anspruch 1, **dadurch gekennzeichnet, dass** eine zumindest teilweise vernetzte Polyacrylsäure verwendet wird.

3. Beschichtungsverfahren nach Anspruch 1 oder 2, **dadurch gekennzeichnet, dass** Polyacrylsäure in einer Flüssigkeit, insbesondere Wasser, dispergiert wird, und die erhaltene Dispersion auf die zu beschichtende Oberfläche aufgebracht wird.

4. Beschichtungsverfahren nach Anspruch 3, **dadurch gekennzeichnet, dass** die erhaltene Dispersion mit einer Base neutralisiert wird.

5. Beschichtungsverfahren nach Anspruch 3 oder 4, **dadurch gekennzeichnet, dass** die Dispersion vor dem Aufbringen auf die zu beschichtende Oberfläche verdünnt wird.

6. Beschichtungsverfahren nach einem der Ansprüche 3 bis 5, **dadurch gekennzeichnet, dass** die Dispersion durch Pipettieren, Sprayen, Dip-Coating-Verfahren oder Spin-Coating-Verfahren aufgebracht wird.

7. Beschichtungsverfahren nach einem der Ansprüche 3 bis 6, **dadurch gekennzeichnet, dass** die Flüssigkeit nach dem Aufbringen der Dispersion durch Erwärmen entfernt wird.

8. Beschichtungsverfahren nach Anspruch 1, **dadurch gekennzeichnet, dass** die zu beschichtende Oberfläche in eine Dextransulfat und/oder Chondroitinsulfat enthaltende wässrige Lösung eingetaucht wird.

9. Beschichtungsverfahren nach Anspruch 8, **dadurch gekennzeichnet, dass** die Substanz durch eine ionische Bindung fest an die Oberfläche gebunden wird.

10. Beschichtungsverfahren nach Anspruch 8 oder 9, **dadurch gekennzeichnet, dass** die Oberfläche nach dem Aufbringen der hydrophilen Schicht vorzugsweise in einem Wasserbad gewaschen wird.

11. Beschichtungsverfahren nach einem der Ansprüche 1 bis 10, **dadurch gekennzeichnet, dass** die beschichtete Oberfläche vorzugsweise in einem Ofen oder Gasstrom getrocknet wird.

12. Beschichtungsverfahren nach einem der Ansprüche 1 bis 11, **dadurch gekennzeichnet, dass** eine zu beschichtende Oberfläche aus einem biokompatiblen Material, insbesondere Edelstahl verwendet wird.

13. Beschichtungsverfahren nach einem der Ansprüche 1 bis 12, **dadurch gekennzeichnet, dass** die beschichtete Oberfläche vorzugsweise durch eine Elektronenstrahlbehandlung sterilisiert wird.

## Claims

1. Coating method for a microfluidic system having a microchannel (12) for the capillary transport of a body fluid for analytical purposes, in which the microchannel (12) is provided with a hydrophilic layer consisting of at least one substance selected from the group polyacrylic acid, polyacrylate, dextran sulfate, chondroitin sulfate, **characterized in that** the substance in a liquid, in particular water, is brought into contact with a surface to be coated and subsequently the liquid is removed, wherein the surface to be coated is cleaned before applying the substance by a oxygen low-pressure plasma method, corona discharge method and/or acid etching method.

2. Coating method according to claim 1, **characterized in that** that an at least partially cross-linked polyacrylic acid is used.

3. Coating method according to claim 1 or 2, **characterized in that** polyacrylic acid is dispersed in a liquid, in particular water, and the dispersion obtained is applied to the surface to be coated.

4. Coating method according to claim 3, **characterized in that** the dispersion obtained is neutralized with a base.

5. Coating method according to claim 3 or 4, **characterized in that** the dispersion is diluted before application to the surface to be coated.

6. Coating method according to one of the claims 3 to 5, **characterized in that** the dispersion is applied by pipetting, spraying, dip-coating methods or spin-coating methods.

7. Coating method according to one of the claims 3 to 6, **characterized in that** the liquid is removed by heating after applying the dispersion.

8. Coating method according to claim 1, **characterized in that** the surface to be coated is immersed in an aqueous solution containing dextran sulfate and/or chondroitin sulfate.

9. Coating method according to claim 8, **characterized in that** the substance is bound firmly to the surface by an ionic bond.

10. Coating method according to claim 8 or 9, **characterized in that** the surface is washed preferably in a water bath after the hydrophilic layer has been applied.

11. Coating method according to one of the claims 1 to 10, **characterized in that** the coated surface is preferably dried in an oven or in a stream of gas.

12. Coating method according to one of the claims 1 to 11, **characterized in that** a surface to be coated made of a biocompatible material and in particular high-grade steel is used.

13. Coating method according to one of the claims 1 to 12, **characterized in that** the coated surface is preferably sterilized by an electron beam treatment.

## Revendications

1. Procédé de revêtement pour un système microfluidique, qui présente un microcanal (12) pour le transport capillaire d'un liquide corporel à des fins d'analyse, dans lequel le microcanal (12) est pourvu d'une couche hydrophile constituée par au moins une substance choisie dans le groupe formé par le poly(acide acrylique), le polyacrylate, le sulfate de dextrane, le sulfate de chondroïtine, **caractérisé en ce que** la substance est amenée, dans un liquide, en particulier de l'eau, en contact avec une surface à revêtir et le liquide est ensuite éliminé, la surface à revêtir étant nettoyée, avant l'application de la substance, par un procédé au plasma basse pression d'oxygène, un procédé de décharge corona et/ou un procédé de décapage à l'acide.

2. Procédé de revêtement selon la revendication 1, **caractérisé en ce qu'**on utilise un poly(acide acrylique) au moins partiellement réticulé.

3. Procédé de revêtement selon la revendication 1 ou 2, **caractérisé en ce que** le poly(acide acrylique) est dispersé dans un liquide, en particulier de l'eau, et la dispersion obtenue est appliquée sur la surface à revêtir.

4. Procédé de revêtement selon la revendication 3, **caractérisé en ce que** la dispersion obtenue est neutralisée par une base.

5. Procédé de revêtement selon la revendication 3 ou 4, **caractérisé en ce que** la dispersion est diluée avant l'application sur la surface à revêtir.

6. Procédé de revêtement selon l'une quelconque des revendications 3 à 5, **caractérisé en ce que** la dispersion est appliquée par pipetage, par pulvérisation, par un procédé de dépôt par trempage ou par un procédé de dépôt à la tournette.

7. Procédé de revêtement selon l'une quelconque des revendications 3 à 6, **caractérisé en ce que** le liquide est éliminé par chauffage après l'application de la dispersion.

8. Procédé de revêtement selon la revendication 1, **caractérisé en ce que** la surface à revêtir est plongée dans une solution aqueuse contenant du sulfate de dextrane et/ou du sulfate de chondroïtine.

9. Procédé de revêtement selon la revendication 8, **caractérisé en ce que** la substance est liée de manière fixe à la surface par une liaison ionique.

10. Procédé de revêtement selon la revendication 8 ou 9, **caractérisé en ce que** la surface est lavée, de préférence dans un bain d'eau, après l'application de la couche hydrophile.

11. Procédé de revêtement selon l'une quelconque des revendications 1 à 10, **caractérisé en ce que** la surface revêtue est séchée, de préférence dans un four ou un flux gazeux.

12. Procédé de revêtement selon l'une quelconque des revendications 1 à 11, **caractérisé en ce qu'**on utilise une surface à revêtir en un matériau biocompatible, en particulier en acier inoxydable.

13. Procédé de revêtement selon l'une quelconque des revendications 1 à 12, **caractérisé en ce que** la surface revêtue est stérilisée, de préférence par un traitement par des rayons électroniques.
